(19) Europäisches Patentamt
European Patent Office
Office européen des brevets

(11) **EP 4 299 145 B1**

(12) **EUROPEAN PATENT SPECIFICATION**

(45) Date of publication and mention
of the grant of the patent:
**16.04.2025 Bulletin 2025/16**

(21) Application number: **23181933.5**

(22) Date of filing: **28.06.2023**

(51) International Patent Classification (IPC):
***A63B 23/18*** *(2006.01)*

(52) Cooperative Patent Classification (CPC):
**A63B 23/185;** A63B 2225/62; A63B 2230/42

(54) **CONTROL OF PACED BREATHING DEVICE**

STEUERUNG EINER ATEMVORRICHTUNG MIT SCHRITT

COMMANDE DE DISPOSITIF RESPIRATOIRE STIMULÉ

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.06.2022 PCT/CN2022/102412**
**02.09.2022 EP 22193701**

(43) Date of publication of application:
**03.01.2024 Bulletin 2024/01**

(73) Proprietor: **Koninklijke Philips N.V.**
**5656 AG Eindhoven (NL)**

(72) Inventors:
• **JIN, Sheng**
**5656 AG Eindhoven (NL)**
• **SHI, Jun**
**5656 AG Eindhoven (NL)**
• **YIN, Bin**
**5656 AG Eindhoven (NL)**

(74) Representative: **Philips Intellectual Property &**
**Standards**
**High Tech Campus 52**
**5656 AG Eindhoven (NL)**

(56) References cited:
**CN-A- 113 926 157     US-A1- 2020 178 887**

**Description**

FIELD OF THE INVENTION

Embodiments of the present disclosure generally relate to a field of breathing device, and more specially, to a method for controlling a paced breathing device, a paced breathing device, and a computer program product.

BACKGROUND OF THE INVENTION

A slow and regular breathing activity is considered beneficial for relaxation. Paced breathing may be used to calm down the body and distract the mind from worries. A paced breathing device may be used to guide a user to perform paced breathing via touching a surface of the device. The surface of the device may fluctuate up and down to simulate a breathing pattern. The breathing pattern may include a series of breathing cycles defined by breathing parameters, and the breathing parameters may be fixed or change over time. The user can follow this breathing pattern to control his/her breathing by tactile sensing.

To control the surface of the paced breathing device up and down with a good sense of touch, an airbag in the paced breathing device may be controlled to inflate and deflate. Generally, an actuator, such as an air pump, is used to pump air into the airbag to inflate the airbag during an inhalation phase of a breathing cycle, and a valve is opened to release the air so as to deflate the airbag during an exhalation phase of the breathing cycle. However, the conventional paced breathing device is not able to provide good breathing experience for users.

SUMMARY OF THE DISCLOSURE

Currently, the flow rate at which the paced breathing device releases air through a valve is uncontrollable, and thus the time length of deflation, which represents the exhalation phase in a breathing cycle, is uncontrollable. During a paced breathing exercise, the time length of each breathing phase of a user and thus his breathing pattern changes when his breathing parameter (e.g. a breathing rate, an exhalation-inhalation ratio, and etc.) changes. Additionally, different users may have different breathing parameter, and thus breathing patterns. This will cause the undulation of the surface of the paced breathing device not to be aligned with the user's breath, which will impact user experience and compliance to follow the exercises

Document CN113926157A discloses a paced breathing device comprising an airbag, a processor a pump and a memory.

In view of the above, embodiments of the present disclosure propose a method, a device and computer program for controlling paced breathing.

**In** a first aspect, the embodiments of the present disclosure provide a method for controlling a paced breathing device. The method comprises determining a flow rate for pumping air into an airbag of the paced breathing device in an exhalation phase of the paced breathing device. The method further comprises pumping, at the determined flow rate, the air into the airbag in the exhalation phase during which the airbag releases the air.

According to embodiments of the present disclosure, the paced breathing device pumps air into the airbag while releasing the air in the exhalation phase. **In** this way, the air volume in the airbag is compensated and the exhalation time length of the exhalation phase can be controlled precisely to be aligned with the user's breathing pattern. This can improve user experience and compliance during the paced breathing exercise.

**In** some embodiments of the first aspect, the flow rate may include at least one compensation flow rate, and determining the at least one compensation flow rate may comprise obtaining an inflation flow rate for pumping the air into the airbag in an inhalation phase of the paced breathing device, and determining the at least one compensation flow rate based on the inflation flow rate, a deflation flow rate at which the airbag releases the air, and breathing parameters. **In** this way, the paced breathing device can precisely determine the compensation flow rate to adjust the exhalation time length of the exhalation phase.

**In** some embodiments of the first aspect, the breathing parameters may include breaths per minute (BPM) for the paced breathing device and user breathing parameters. The user breathing parameters may include an inhalation hold time length, an exhalation hold time length and an exhalation-to-inhalation ratio. Determining the at least one compensation flow rate may comprise determining a time length of a breathing cycle based on the BPM, and determining an inhalation time length of the inhalation phase and an exhalation time length of the exhalation phase based on the time length of the breathing cycle, the exhalation-to-inhalation ratio, the inhalation hold time length and the exhalation hold time length, and determining the at least one compensation flow rate based on the inhalation time length, the inflation flow rate, the exhalation time length and the deflation flow rate. In this way, the paced breathing device can determine the compensation flow rate according to user-specific breathing characteristics and thus can align the time length of the breathing cycle with the user's breathing pattern.

In some embodiments of the first aspect, the exhalation phase may include N stages over time, where *N* is an integer greater than or equal to 2. Determining the at least one compensation flow rate may further comprise determining a compensation flow rate for each stage of the N stages respectively. In this way, the paced breathing device can generate a breathing pattern more similar to a nature breathing waveform of a user.

In some embodiments of the first aspect, the method may further comprise dividing the exhalation phase into the N stages based on user breathing characteristics. In this way, the paced breathing device can generate user-specific and precise breathing patterns for users.

In some embodiments of the first aspect, the user breathing parameters may further include a first ratio indicating a proportion of a time length of a stage of the N stages to the exhalation time length of the exhalation phase, and a second ratio indicating a proportion of volume to be released in the airbag during the stage to volume to be released in the airbag during the exhalation phase. Determining a compensation flow rate for each stage of the N stages respectively may comprise determining a compensation flow rate for each stage of the N stages based on the inhalation time length, the inflation flow rate, the exhalation time length, the deflation flow rate, the first ratio and the second ratio. In this way, the paced breathing device can determine the compensation flow rate for pumping into the airbag for each stage of the exhalation phase.

In some embodiments of the first aspect, the method may further comprise determining the user breathing parameters based on one or more of: a user interaction with the paced breathing device, and a predefined setting of the paced breathing device according to an analysis of historical breathing characteristics of multiple users. In this way, the paced breathing device can determine the compensation flow rate to generate a breathing pattern compatible with the user's breath pattern.

In some embodiments of the first aspect, the method may further comprise adjusting a duty cycle of Power Width Modulation (PWM) of an actuator of the paced breathing device to enable the determined flow rate. In this way, the paced breathing device may easily and conveniently implement the compensation flow rate with the actuator.

In some embodiments of the first aspect, the method may further comprise pumping, at the determined flow rate, the air into the airbag whiling releasing the air in exhalation phases repeatedly. In this way, the paced breathing device may repeatedly fluctuate up and down to provide a high user experience consistently during each breathing cycle of the breathing pattern of the paced breathing device.

In a second aspect, the embodiments of the present disclosure provide a paced breathing device. The paced breathing device comprises an airbag, a processor and a memory having a plurality of instructions stored thereon. The instructions, when executed by the processor, cause the device to determine a flow rate for pumping air into the airbag in an exhalation phase of the paced breathing device. The instructions further cause the device to pump, at the determined flow rate, the air into the airbag in the exhalation phase during which the airbag releases the air.

The embodiments of the second aspect may achieve similar advantages and technical effect as the first aspect, which are thus omitted for simplicity.

In some embodiments of the second aspect, the flow rate may include at least one compensation flow rate. The instructions, when executed, may further cause the device to obtain an inflation flow rate for pumping the air into the airbag in an inhalation phase of the paced breathing device, and determine the at least one compensation flow rate based on the inflation flow rate, a deflation flow rate at which the airbag releases the air, and breathing parameters.

**In** some embodiments of the second aspect, the breathing parameters may include BPM for the paced breathing device and user breathing parameters. The user breathing parameters may include an inhalation hold time length, an exhalation hold time length, and an exhalation-to-inhalation ratio. The instructions, when executed, may further cause the device to determine a time length of a breathing cycle based on the BPM, determine an inhalation time length of the inhalation phase and an exhalation time length of the exhalation phase based on the time length of the breathing cycle, the exhalation-to-inhalation ratio, the inhalation hold time length and the exhalation hold time length, and determine the at least one compensation flow rate based on the inhalation time length, the inflation flow rate, the exhalation time length and the deflation flow rate.

In some embodiments of the second aspect, the exhalation phase may include N stages over time, where N is an integer greater than or equal to 2. The instructions, when executed, may further cause the device to determine a compensation flow rate for each stage of the N stages respectively.

In some embodiments of the second aspect, the instructions, when executed, further cause the device to divide the exhalation phase into the N stages based on user breathing characteristics.

In some embodiments of the second aspect, the user breathing parameters further include a first ratio indicating a proportion of a time length of a stage of the N stages to the exhalation time length of the exhalation phase, and a second ratio indicating a proportion of volume to be released in the airbag during the stage to volume to be released in the airbag during the exhalation phase. The instructions, when executed, may further cause the device to determine a compensation flow rate for each stage of the N stages based on the inhalation time length, the inflation flow rate, the exhalation time length, the deflation flow rate, the first ratio and the second ratio.

In some embodiments of the second aspect, the instructions, when executed, further cause the device to determine the user breathing parameters based on one or more of a user interaction with the paced breathing device and a predefined setting of the paced breathing device according to an analysis of historical breathing characteristics of multiple users.

In some embodiments of the second aspect, the instructions, when executed, may further cause the device to adjust a duty cycle of PWM of an actuator of the paced breathing device to enable the determined flow rate.

In some embodiments of the second aspect, the instructions, when executed, may further cause the device to pump at the determined flow rate, the air into the airbag whiling releasing the air in exhalation phases repeatedly along with the user's breathing pattern.

In some embodiments of the second aspect, the device may be a hugging pillow.

In a third aspect, the embodiments of the present disclosure provide a computer program product comprising a computer readable medium. The computer readable medium has computer readable code embodied therein. The computer readable code is configured such that, on execution by the processor of the paced breathing device according to the second aspect, the paced breathing device is caused to perform the method of the first aspect.

It is to be understood that the Summary is not intended to identify key or essential features of embodiments of the present disclosure, nor is it intended to be used to limit the scope of the present disclosure. Other features of the present disclosure will become easily comprehensible through the description below.

BRIEF DESCRIPTION OF THE DRAWINGS

The details of one or more embodiments of the present disclosure are set forth in the accompanying drawings and the description below. Other features, aspects, and advantages of the disclosure will become apparent from the description, the drawings, and the claims, wherein:

Fig. 1 is a block diagram illustrating an example paced breathing device in accordance with an example embodiment of the present disclosure;
Fig. 2 is a graph illustrating an example breathing cycle of the example paced breathing device of Fig. 1;
Fig. 3 is a flowchart illustrating an example process for controlling a paced breathing device in accordance with an example embodiment of the present disclosure;
Fig. 4 is a flowchart illustrating an example process for determining a compensation flow rate in an exhalation phase in accordance with an example embodiment of the present disclosure;
Fig. 5A is a graph illustrating an example of the air volume in the airbag during a breathing cycle at a first BPM;
Fig. 5B is a graph illustrating an example of an air flow rate of pumping in during the breathing cycle at the first BPM;
Fig. 6A is a graph illustrating another example of the air volume in the airbag during a breathing cycle at a second BPM;
Fig. 6B is a graph illustrating another example of an air flow rate of pumping in during the breathing cycle at the second BPM;
Fig. 7 is a flowchart illustrating an example process for determining compensation flow rates for multiple stages of the exhalation phase in accordance with an example embodiment of the present disclosure;
Fig. 8A is a graph illustrating an example of the air volume in the airbag during a breathing cycle, the exhalation phase of which has multiple stages; and
Fig. 8B is graph illustrating an example of air flow rates of pumping in during the breathing cycle, the exhalation phase of which has multiple stages.

Throughout the figures, same or similar reference numbers will always indicate same or similar elements.

DETAILED DESCRIPTION OF EMBODIMENTS

Principle of the present disclosure will now be described with reference to some example embodiments. It is to be understood that these embodiments are described only for the purpose of illustration and help those skilled in the art to understand and implement the present disclosure, without suggesting any limitations as to the scope of the disclosure. The disclosure described herein can be implemented in various manners other than the ones describe below.

As used herein, the term "comprise/include" and its variants are to be read as open terms that mean "comprise/include, but not limited to." The term "based on" is to be read as "based at least in part on." The term "one embodiment" and "an embodiment" are to be read as "at least one embodiment." The term "another embodiment" is to be read as "at least one other embodiment." Moreover, it is to be understood that in the context of the present disclosure, the terms "first," "second" and the like are used to indicate individual elements or components, without suggesting any limitation as to the order of these elements. Further, a first element may or may not be the same as a second element.

As mentioned above, to control a surface of a paced breathing device up and down with a good sense of touch, the paced breathing device pumps air into an airbag in an inhalation phase of a breathing cycle. When an exhalation phase comes, the paced breathing device stops pumping, and opens a valve to release the air in the airbag. Due to the uncontrollable releasing air flow rate via the valve, the exhalation time length of the exhalation phase cannot be controlled to meet the requirements of various breathing patterns of different users.

A method addresses this problem by quickly switching the valve on and off to control the overall releasing flow rate. However, frequently switching shortens the lifetime of the valve. Moreover, the paced breathing device is generally used to relax and help user fall asleep smoothly, but the noises produced by the valve are undesirable, and thus user experience is degraded. To address these and other potential problems, embodiments of the present disclosure provide a solution for controlling the paced breathing device.

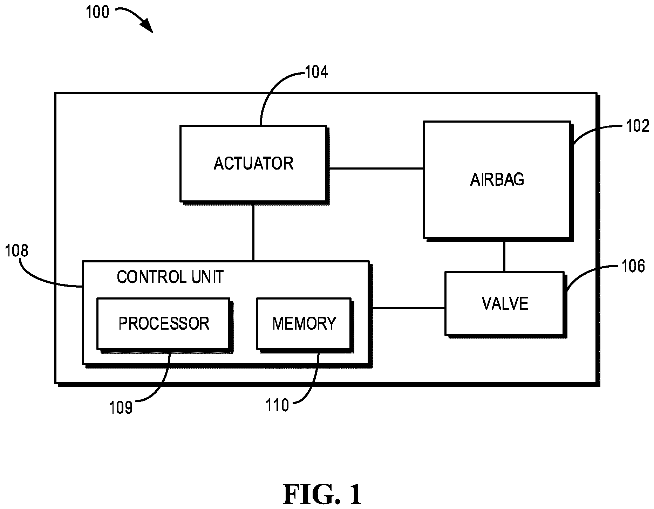

Reference is now made to Fig. 1 which illustrates an example paced breathing device 100 in accordance with an example embodiment of the present disclosure. The paced breathing device 100 may be implemented as a tactile breathing device, for example, a hugging pillow or the like. The surface of the paced breathing device 100 may fluctuate up and down according to a breathing pattern programmed in the device. The breathing pattern may include a series of breathing cycles. Each of the breathing cycles is defined by breathing parameters, and breathing parameters in different breathing cycles may be the same or different. A user can follow this breathing pattern to control his/her breathing by tactile sensing while hugging the pillow.

As shown, the paced breathing device 100 includes an airbag 102, an actuator 104, a valve 106, and control unit 108, and the control unit 108 may include a processor 109 and a memory 110. It is to be understood that although the control unit 108 is shown in Fig. 1, the control unit 108 may be omitted from the paced breathing device 100, and the combination of the processor 109 and the memory 110 may perform the control operations for the paced breathing device 100. The airbag 102 may be made of airtight and elasticity material such that it inflates when the air volume in the airbag 102 increases and deflates when the air volume decreases. The actuator 104 is coupled, for example, directly or via a duct, to the airbag 102 and operates to increase the air volume in the airbag. In some embodiments, the actuator 104 may be an air pump, for example. The actuator 104 may pump the air flow into the airbag at a time interval and at a flow rate according to control signals transmitted from the control unit 108.

The valve 106 is coupled, for example, directly or via a duct, to the airbag 102 and operates to decrease the air volume in the airbag 102 by releasing the air. For example, the valve 106 may be an electromagnetic valve which can be open and closed according to control signals transmitted from the control unit 108.

With the valve 106 being closed and the actuator 104 pumping air into the airbag 102, the air volume in the airbag 102 increases and the surface of the paced breathing device 100 fluctuates up. With the valve 106 being open, the air volume in the airbag 102 decreases and the surface of the paced breathing device 100 fluctuates down. In other words, the control unit 108 controls the valve 106 and actuator 104 to enable the undulation of the surface of the paced breathing device 100.

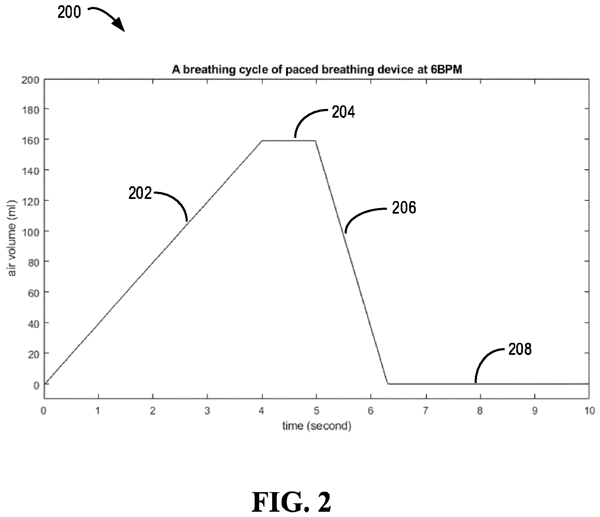

In some embodiments, the control unit 108 may control the paced breathing device 100 according to breathing patterns (not shown) programmed in the memory 110. The breathing patterns are indicative of how the paced breathing device 100 guides the user to breathe, for example, when to exhale and when to inhale. The breathing pattern may be defined by various breathing parameters, for example breathes per minute (BPM) and user breathing parameters. The breathing parameters will be described in detail with reference to Fig. 2. The control unit 108 may transmit corresponding control signals to the actuator 104 and the valve 106 to enable desired breathing parameters, and thus enable compatibility between the breathing patterns of the user and the device.

Although the example paced breathing device 100 is shown and described with reference to Fig. 1, it is to be understood and appreciated that the paced breathing device is not limited by the above description with reference to Fig. 1. It is to be understood and appreciated that the paced breathing device according to the disclosure may comprise additional components that are not shown, and some of the components in Fig. 1 may be omitted. For example, the paced breathing device may further include various sensors to detect user breathing parameters to simulate user-specific breathing patterns and to improve user experience. The paced breathing device may further include input/output interfaces for users to operate. For example, a user may input his individual breathing parameters via the input interface to the paced breathing device.

Fig. 2 is a graph illustrating an example breathing cycle 200 of the example paced breathing device 100 of Fig. 1. The horizontal axis represents a time of the breathing cycle, and the vertical axis represents the volume of air in the airbag 102. As shown in Fig. 2, the breathing rate is 6 BPM, and the time length of the breathing cycle 200 is 10 seconds. In some embodiments, the BPM may be associated with a breathing pattern stored in the paced breathing device 100. Alternatively, the time length of the breathing cycle of the breathing pattern may be stored in the device instead of the BPM. In some embodiments, a user may select a suitable BPM for the paced breathing device 100 when performing his/her paced breathing exercise.

As shown, the breathing cycle 200 includes a plurality of breathing phases. The example breathing cycle 200 starts with an inhalation phase 202, followed by an inhalation hold phase 204, then an exhalation phase 206, and ends with an exhalation hold phase 208.

In this instance, the inhalation phase 202 lasts approximately 4 seconds for example. In the inhalation phase 202, by pumping air into the airbag 102 with the valve 106 closed, the airbag 102 inflates and the air volume in the airbag 102 increases from 0 ml to for example 160 ml. In the inhalation phase 202, the corresponding inflation flow rate for pumping the air into the airbag 102 is approximately 40 ml/second. In some embodiments, the inflation flow rate may be fixed for different breathing cycles. In this case, the total volume of air that is pumped into the airbag 102 depends on the inhalation time length of the inhalation phase 202. Alternatively, the inflation flow rate may vary for different breathing cycles. For example, with a fixed total volume, the paced breathing device 100 may increase the inflation flow rate for shorter inhalation phase and decrease the inflation flow rate for longer inhalation phase. In both cases, the total volume shall not be larger than the maximum volume allowed by the airbag 102 which may depend on the size and material of the airbag 102.

Then, in the inhalation hold phase 204, the paced breathing device 100 stops pumping the air while keeping the valve 106 closed. The air volume in the airbag 102 does not change over time in this phase, and thus the surface of the paced breathing device 100 is kept at a higher position. As shown in Fig.2, the inhalation hold phase 204 lasts approximately 1 second.

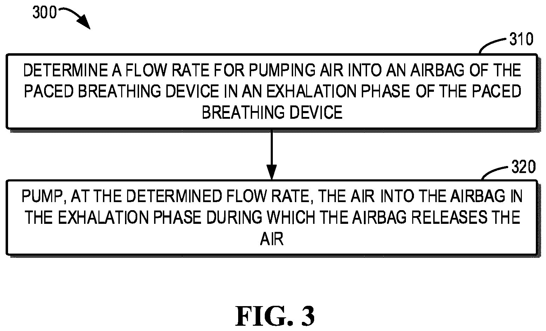

Next, in the exhalation phase 206, the valve 106 of the paced breathing device 100 is open under the control of the control unit 108 to deflate the airbag 102. In this phase, the actuator 104 does not operate, and the air runs out of the airbag 102 at a fixed flow rate via the valve 106. As used herein, the flow rate that the valve 106 releases the air is also referred as deflation flow rate. The exhalation time length of the exhalation phase 206 depends on the total volume in the airbag 102 and the fixed deflation flow rate, for example, 120 ml/second. In some embodiments, the exhalation time length of the exhalation phase 206 can be adjusted by compensation for the air in the airbag 102. This will be described in detail with reference to Figs. 3 to 8B.

At the end of breathing cycle 200, the exhalation hold phase 208 lasts approximately 4 seconds. In this phase, the airbag 102 is empty, and the valve 106 is closed.

While the example breathing cycle 200 is shown and described with reference to Fig. 2, it is to be understood and appreciated that the paced breathing cycle is not limited by the above description. It is to be understood and appreciated that the breathing cycle of the paced breathing device may have various phases and time lengths of the phases. For example, the breathing cycle may exclude one or more of the inhalation hold phase and exhalation hold phase. In addition, the time length of each phase and the time length of the breathing cycle as shown in Fig. 2 are not intended to limit the embodiments of the disclosure.

Fig. 3 is a flowchart illustrating an example process 300 for controlling a paced breathing device in accordance with an example embodiment of the present disclosure. While the methodologies are shown and described as being a series of acts that are performed in a sequence, it is to be understood and appreciated that the methodologies are not limited by the order of the sequence. For example, some acts can occur in a different order than what is described herein. In addition, an act can occur concurrently with another act. Further, in some instances, not all acts may be required to implement a methodology described herein.

Moreover, the acts described herein may be computer-executable instructions that can be implemented by one or more processors and/or stored on a computer-readable medium or media. The computer-executable instructions may include a routine, a sub-routine, programs, a thread of execution, and/or the like. Still further, results of acts of the methodologies may be stored in a computer-readable medium, displayed on a display device, and/or the like. In one embodiment, the process 300 may be implemented by the paced breathing device 100, in particular, by the processor 109 as shown in Fig. 1. For understanding of embodiments of the disclosure, Fig. 3 is described with reference to Figs. 1 and 2.

The paced breathing device 100 may output a suitable breathing pattern in a pre-defined time to guide the user to follow. The breathing pattern may include a series of breathing cycles defined by breathing parameters, for example, a breathing rate. The breathing rate may be a fixed value like 6 breaths per minute (BPM). Alternatively, the breathing rate may be a gradually slowing down breathing rate for example from 12 BPM to 6 BPM, which are in range of normal breathing rates, and then may be kept at 6 BPM until the paced breathing program stops.

As noted above, the paced breathing device 100 pumps air into the airbag in the inhalation phase of a breathing cycle, and releases the air in the airbag in the exhalation phase. In some embodiments of the disclosure, the paced breathing device 100 can control the exhalation time length of the exhalation phase by pumping air in the exhalation phase to compensate the air that is uncontrollably and quickly released via the valve 106.

At block 310, the paced breathing device 100 determines a flow rate for pumping air into an airbag 102 of the paced breathing device 100 in an exhalation phase of the paced breathing device 100. As used herein, the flow rate for pumping air in the exhalation phase may be referred as a compensation flow rate.

In some embodiments, the compensation flow rate may be calculated from the following equation:

$$Fr\_compensation = Fr\_deflation - (T\_inhalation * Fr\_inhalation / T\_exhalation) \quad (1)$$

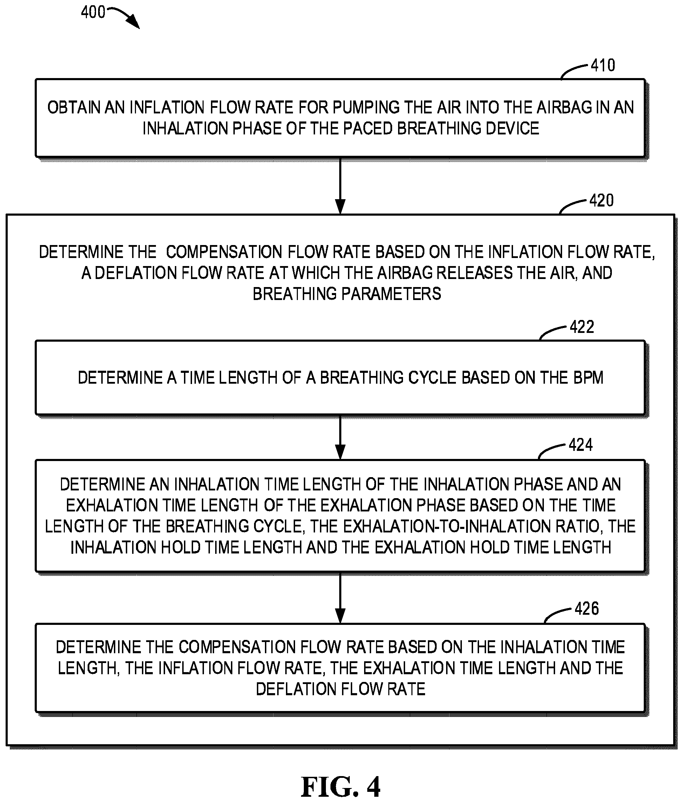

where *Fr_compensation* denotes the compensation flow rate during the exhalation phase, *Fr_deflation* denotes the deflation flow rate at which the airbag releases the air during the exhalation phase, *T inhalation* denotes the inflation time length of the inflation phase, *Fr_inhalation* denotes the inflation flow rate for pumping the air into the airbag during the

inhalation phase, and *Texhalation* denotes the exhalation time length of the exhalation phase. In equation (1), *Fr_deflation* and *Fr_inhalation* may be predefined, and *T_inhalation* and *Texhalation* may be calculated by the paced breathing device 100 according to the BPM and user breathing parameters. Examples of the calculation will be described below with reference to Fig. 4. With equation (1), the paced breathing device 100 may calculate the compensation flow rate by performing a process illustrated in Fig. 4.

At block 320, the paced breathing device 100 pumps, at the determined flow rate, the air into the airbag 102 in the exhalation phase during which the airbag releases the air. For example, in the exhalation phase, the airbag 102 releases the air via the valve, and the air is simultaneously pumped into the airbag at the determined flow rate. In some embodiments, the paced breathing device 100 may adjust a duty cycle of Power Width Modulation (PWM) of the actuator 104 of the paced breathing device 100 to enable the determined flow rate. For instance, if the inflation flow rate during the inhalation phase is 40 ml/second and the corresponding duty cycle is 0.1, then for a required compensation flow rate of 60 ml/second during the exhalation phase, the duty cycle may be adjusted to 0.15. The period of each cycle (frequency of the PWM signal) also can be controlled together with the duty cycle depending on the requirements for a certain actuator.

The paced breathing device 100 may repeatedly pumps the air into the airbag at the determined flow rate whiling releasing the air in the exhalation phases, so as to cause undulation of the paced breathing device 100 repeatedly and compatible with the user's breathing pattern. In some embodiments, the compensation flow rate may be calculated for each breathing cycle, repeatedly. Alternatively, the compensation flow rate may be calculated if the BPM changes in the next breathing cycle. In some embodiments, when the user feels difficult to follow the paced breathing exercise, the compensation flow rate may be adjusted according to a user input of his/her user breathing parameters.

Fig. 4 is a flowchart illustrating an example process 400 for determining a compensation flow rate in an exhalation phase in accordance with an example embodiment of the present disclosure. The process 400 is an example implementation of block 310 in Fig. 3.

At block 410, the paced breathing device 100 obtains an inflation flow rate for pumping the air into the airbag in an inhalation phase of the paced breathing device. The inflation flow rate may be fixed and programmed in the control unit 108, or may be determined on the fly based on the inhalation time length of the inhalation phase.

At block 420, the paced breathing device 100 determines the compensation flow rate based on the inflation flow rate, a deflation flow rate at which the airbag releases the air, and breathing parameters. The breathing parameters may indicate time information of each phase of the breathing cycle.

The breathing parameters may include the BPM and user breathing parameters. The user breathing parameters may include an exhalation-to-inhalation ratio which indicates a proportion of a time length of the exhalation phase to a time length of the inhalation phase. Alternatively, the user parameters may include an inhalation-exhalation ratio which is reciprocal of the exhalation-to-inhalation ratio. In case of the breathing cycle including hold phases, the user breathing parameters may further include an inhalation hold time length and an exhalation hold time length. In this case, the exhalation-to-inhalation ratio may indicate a ratio of the exhalation time length of the exhalation phase plus the exhalation hold time length of the exhalation hold phase to the inhalation time length of the inhalation phase plus the inhalation hold time length of the inhalation hold phase. It is to be understood that inhalation hold time length of value 0 implies that the inhalation hold phase does not exist and exhalation hold time length of value 0 implies that the exhalation hold phase does not exist.

In some embodiments, the user breathing parameters may be determined based on user interactions with the paced breathing device 100. For example, the user may input his/her breathing parameters to the paced breathing device 100 prior to using the paced breathing device 100. As another example, the paced breathing device 100 may collect the user's breathing data through one or more sensors to obtain the user breathing parameters. The breathing data may include various physiological and behavior data when the user performs the paced breathing exercise and/or the user breathes in his daily life. In some embodiments, the user's breathing parameters may also be determined based on predefined settings of the paced breathing device, which may had been derived according to an analysis of historical breathing data of multiple users.

Referring to Fig. 4, to determine the compensation flow rate, at block 422, the paced breathing device 100 may determine a time length of a breathing cycle based on the BPM. For example, the time length of a breathing cycle may be calculated from the following equation:

$$T\_breath\_cycle = 60 / Br\_paced\_breathing\_breath\_rate \qquad (2)$$

where *T_breath_cycle* denotes a time length of a breathing cycle, and *Br_paced_breathing_breath_rate* denotes the BPM.

At block 424, the paced breathing device 100 may determine an inhalation time length of the inhalation phase and an exhalation time length of the exhalation phase based on the time length of the breathing cycle, the exhalation-to-inhalation ratio, the inhalation hold time length and the exhalation hold time length.

As an example, the inhalation time length of the inhalation phase may be calculated from the following equation:

$$T\_inhalation = T\_breath\_cycle/(R\_exhalation\_to\_inhalation + 1) - T\_inhalation\_hold$$

$$(3)$$

where *T_inhalation* denotes the inhalation time length of the inhalation phase, *T_breath_cycle* denotes the time length of the breathing cycle, *R_exhalation_to_inhalatio* denotes the exhalation-to-inhalation ratio, and *T_inhalation_hold* denotes the inhalation hold time length. Further, the exhalation time length of the exhalation phase may be calculated from the follow equation:

$$T\_exhalation = T\_breath\_cycle - T\_inhalation - T\_inhalation\_hold - T\_exhalation\_hold$$

$$(4)$$

where *Texhalation* denotes the exhalation time length of the exhalation phase, *T_breath_cycle* denotes the time length of the breathing cycle, *T_inhalation_hold* denotes the inhalation hold time length, and *T_exhalation_hold* denotes the exhalation hold time length.

Alternatively, the exhalation time length of the exhalation phase may be determined first, and the inhalation time length of the inhalation phase may be determined according to the determined time length of the exhalation phase.

At block 426, the paced breathing device 100 determines the compensation flow rate based on the inhalation time length, the inflation flow rate, the exhalation time length and the deflation flow rate. In some embodiments, the paced breathing device 100 may use equation (1) to calculate the compensation flow rate.

According to embodiments of the disclosure, the exhalation time length of exhalation phase can be controlled precisely hence to be aligned with the user's breathing pattern. This can improve user experience and compliance during the paced breathing exercise.

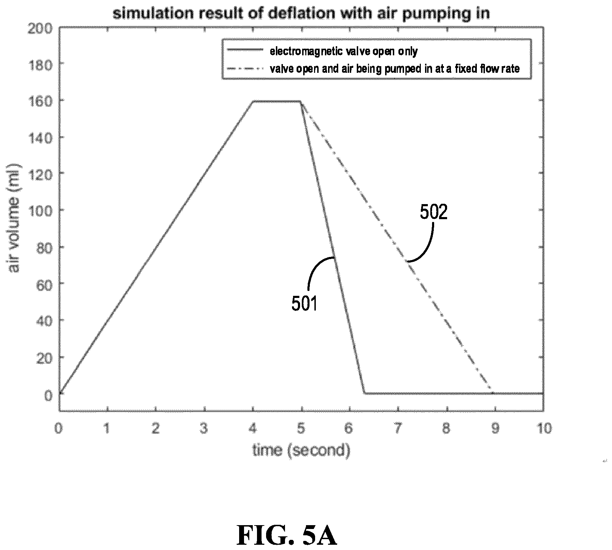

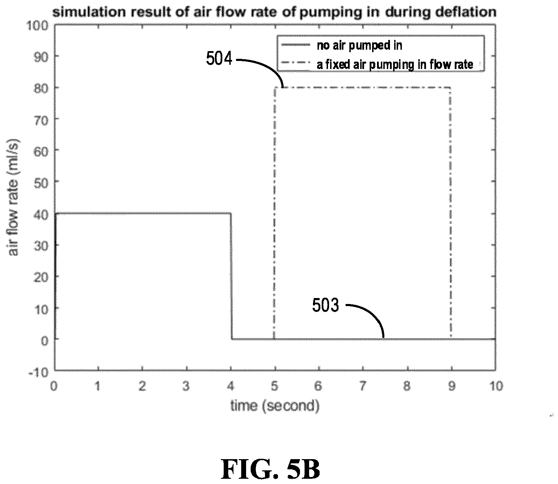

Fig. 5A is a graph illustrating an example of air volume in the airbag during a breathing cycle at a first BPM, and Fig. 5B is a graph illustrating an example of an air flow rate of pumping in during a breathing cycle at the first BPM.

The example in Fig. 5A has a breathing rate of 6 BPM, inflation flow rate of 40 ml/second, deflation flow rate of 120 ml/second. In Fig. 5A, the solid line 501 represents the air volume in the airbag over time where the electromagnetic valve opens only during the exhalation phase. Without compensation of the air pump, the air volume in the airbag decreases quickly in the exhalation phase and arrives at the bottom in about 1.3 second.

Given a paced breathing cycle with a ratio of an exhalation phase time length (exhalation time length + exhalation hold time length) to an inhalation phase time length (inhalation time length + inhalation hold time length) equal to 1, an inhalation hold time length of 1 second and an exhalation hold time length of 1 second, then the compensation flow rate is calculated to be equal to about 80 ml/second. The dash-dotted line 502 represents air volume in the airbag in the exhalation phase of the given breathing cycle. By pumping air into the airbag, the exhalation time length of the exhalation phase is under control and extended to 4 seconds.

In Fig. 5B, the solid line 503 represents the air flow rate of pumping over time where the electromagnetic valve opens only during the exhalation phase. In the inhalation phase, the air is pumped into the airbag at the flow rate of 40 ml/second, and in the exhalation phase, there is no air being pumped in. The dash-dotted line 504 represents the air flow rate of pumping where the electromagnetic valve opens with a fixed flow rate of air pumping in during exhalation phase. The paced breathing device pumps the air at 80 ml/second as compensation and extends the exhalation time length of the exhalation phase.

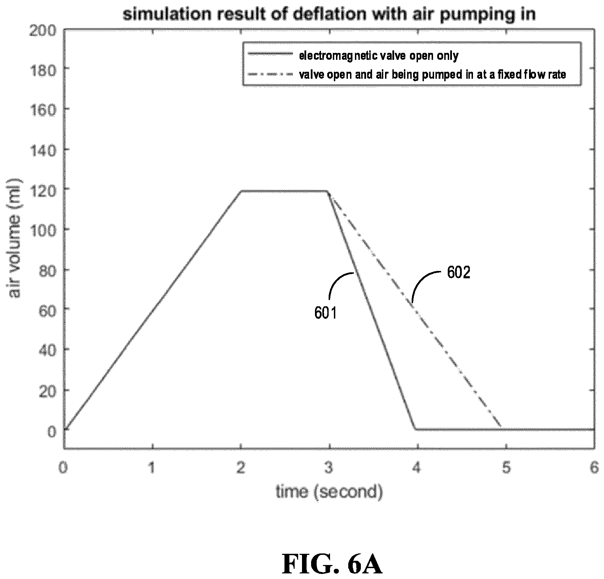

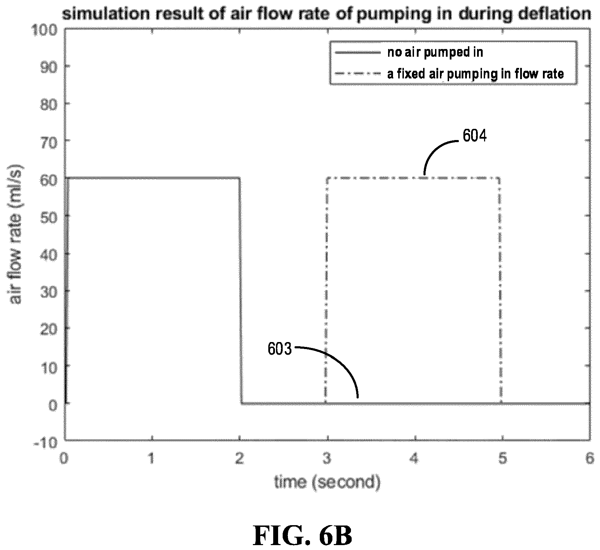

Fig. 6A is a graph illustrating another example of air volume in the airbag during a breathing cycle at a second BPM, and Fig. 6B is a graph illustrating an example of an air flow rate of pumping in during a breathing cycle at the second BPM.

The example in Fig. 6A has a breathing rate of 10 BPM, an inflation flow rate of 60 ml/second, a deflation flow rate of 120 ml/second. **In** Fig. 6A, the solid line 601 represents the air volume in the airbag over time where the electromagnetic valve opens only during the exhalation phase. Without compensation, the air volume in the airbag decreases quickly in the exhalation phase and arrives at the bottom in about 1 second.

Given another paced breathing cycle with a ratio of an exhalation phase time length (exhalation time length + exhalation hold time length) to an inhalation phase time length (inhalation time length + inhalation hold time length) equal to 1, an inhalation hold time length of 1 second and an exhalation hold time length of 1 second, then the compensation flow rate is calculated to be equal to about 60 ml/second. The dash-dotted line 602 represents the air volume in the airbag in the exhalation phase of the given breathing cycle. By pumping the air into the airbag, the exhalation time length of the exhalation phase is under control and extended from 1 second to 2 seconds.

In Fig. 6B, the solid line 603 represents the air flow rate of pumping over time where the electromagnetic valve opens only during the exhalation phase. In the inhalation phase, the air is pumped into the airbag at the flow rate of 60 ml/second, and in the exhalation phase, there is no air being pumped in. The dash-dotted line 604 represents the air flow rate of pumping where the electromagnetic valve opens with a fixed flow rate of air pumping in during the exhalation phase. The paced breathing device pumps the air at 60 ml/second as compensation and extends the exhalation time length of the exhalation phase.

It is to be understood that the numbers used in examples described with reference to Figs. 5A, 5B, 6A, and 6B are just for instance without limiting the disclosure.

In the embodiments described with reference to Fig. 3 to Fig. 6B, the paced breathing device pumps air at a fixed flow rate over the whole exhalation phase. Considering that the nature breathing waveform of expiration of human being may be a non-linear curve that the beginning phase of air flow rate of expiration is larger than the rest phases, embodiments of the disclosure further control the airbag surface deflating down to simulate this breathing waveform. In some embodiments, the exhalation phase may be divided into multiple stages, and compensation flow rate may be calculated for each stage.

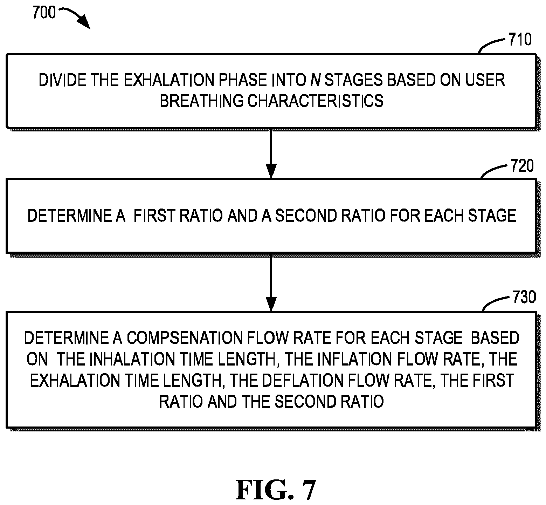

Fig. 7 is a flowchart illustrating an example process 700 for determining compensation flow rates for multiple stages of the exhalation phase in accordance with an example embodiment of the present disclosure.

At block 710, the paced breathing device divides the exhalation phase into N stages based on user breathing characteristics, wherein N is an integer greater than or equal to 2. The user breathing characteristics may be obtained by sensing breathing data of the current user. Alternatively, a user can actively input the breathing data into the paced breathing devices. In some embodiments, the user breathing characteristics may be obtained by collecting breathing data of multiple users and may be preconfigured in the paced breathing device. From the user breathing characteristics, a nature breathing waveform may be plotted as a nonlinear curve. By a curve-fitting method, multiple stages may be calculated to approximate the nonlinear curve, and time information about stages may be used to divide the exhalation phase into multiple stages.

At block 720, the paced breathing device 100 determines a first ratio and a second ratio for each stage. The first ratio indicates a proportion of a time length of a stage of the N stages to the exhalation time length of the exhalation phase. The second ratio indicates a proportion of volume to be released in the airbag during the stage to volume to be released in the airbag during the exhalation phase. In other words, the first ratio measures the relative time length that one stage occupies the exhalation phase, and the second ratio measures a relative volume of air to be released during the stage compared with the total air volume to be released in the exhalation phase. The first ratio and the second ratio may be in range of 0-1, inclusive. A sum of the first ratios for all stages respectively is equal to 1. A sum of the second ratios for all stages respectively is equal to 1. The first ratio and the second ratio in each stage is determined based on the user breathing characteristics so as to facilitate the alignment between the breathing patterns of the paced breathing device and the user. It is to be understood that the acts at block 710 and block 720 can occur simultaneously or in a different order than what is described herein. Based on the multiple stages and the calculated ratios for each stage, the paced breathing device may generate a breathing cycle, which is compatible with a nature breathing waveform of a user, and thus generate a breathing pattern that is more precisely aligned with user's breathing pattern. Thereby, the user feels more comfortable in the paced breathing exercise.

At block 730, the paced breathing device 100 determines a compensation flow rate for each stage based on the inhalation time length, the inflation flow rate, the exhalation time length, the deflation flow rate, the first ratio and the second ratio. The compensation flow rate for the i-th stage may be calculated from the equation as below:

$$Fr\_compensation\_i = Fr\_deflation - \frac{T\_inhalation * Fr\_inhalation * ratio1\_i}{T\_exhalation * ratio2\_i} \quad (5)$$

where $Fr\_compensation\_i$ denotes the compensation flow rate for the i-th stage of the N stages, $Fr\_deflation$ denotes the deflation flow rate at which the airbag releases the air during the exhalation phase, $T\ inhalation$ denotes the inflation time length of the inflation phase, $Fr\_inhalation$ denotes the inflation flow rate, and $T\ exhalation$ denotes the exhalation time length of the exhalation phase, $ratio1\_i$ denotes the first ratio for the i-th stage, and $ratio2\ i$ denotes the second ratio for the i-th stage. Here, $Fr\_deflation, Fr\_inhalation, T\ inhalation,$ and $T exhalation$ may be calculated according to the embodiments described with reference to Figs. 3 and 4.

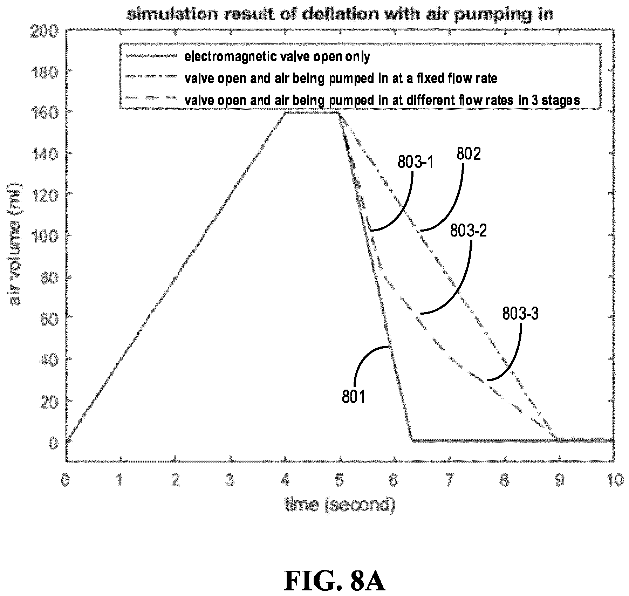

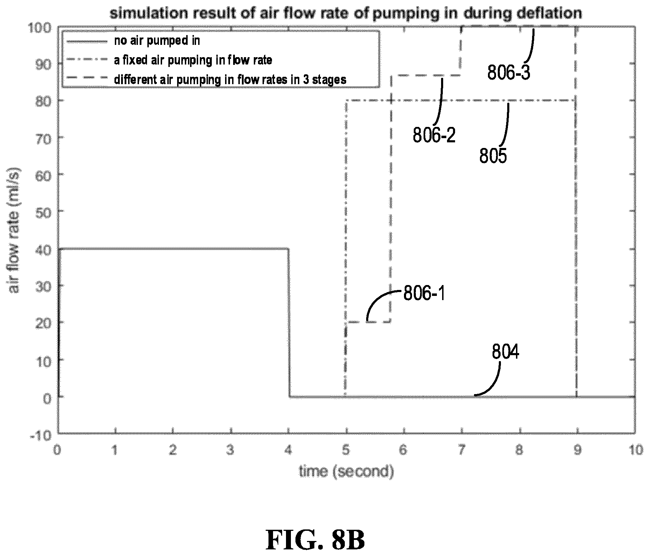

Fig. 8A is a graph illustrating an example of the air volume in the airbag during a breathing cycle, the exhalation stage of which has multiple stages. Fig. 8B is graph illustrating an example of air flow rates of pumping in during the breathing cycle, the exhalation stage of which has multiple stages. Although the exhalation phase in Figs. 8A and 8B comprises three stages, it is to be understood that the exhalation phase may comprise any number of stages that are matched with the user's breathing pattern for better user experience.

The example in Fig. 8A has a breathing rate of 6 BPM, an inflation flow rate of 40 ml/second, deflation flow rate of 120 ml/second. The paced breathing cycle in Fig. 8A has an exhalation phase time length (exhalation time length + exhalation hold time length) to inhalation phase time length (inhalation time length + inhalation hold time length) ratio of 1, an inhalation hold time length of 1 second and an exhalation hold time length of 1 second, as in Figs. 5A and 5B.

In Fig. 8A, the solid line 801 represents the air volume in the airbag over time where the electromagnetic valve opens without compensation during the exhalation phase. Similar to Fig. 5A, the solid line indicates that the air volume in the airbag decreases quickly and arrives at the bottom in about 1.3 second. The dash-dotted line 802 represents the air volume where the electromagnetic valve opens with a fixed flow rate of air being pumped in during the exhalation phase. Similar to Fig. 5A, the dash-dotted line indicates that, by way of the fixed compensation flow rate, the exhalation time length of the exhalation phase is extended from 1.3 second to 4 seconds.

The dashed lines 803-1, 803-2, and 803-3 represent the air volume over time where the electromagnetic valve opens and the air is pumped in at different flow rates for three stages of the exhalation phase. As an example, the ratios for the stages 1 to 3 are as below:

Stage 1: ratio1_1 = 0.2, ratio1_2 = 0.5;
Stage 2: ratio1_2 = 0.3, ratio2_2 = 0.25;
Stage 3: ratio1_3 = 0.5, ratio2_3 = 0.25;

That is, the time length of Stage 1 is 20% of the exhalation time length, the volume to be released for Stage 1 is 50% of the total air volume to be released; the time length of Stage 2 is 30% of exhalation time length, the volume decreases for Stage 803-2 is 25% of the total air volume to be released; and the time length of Stage 3 is 50% of exhalation time length, the volume decreases for Stage 3 is 25% of the total air volume to be released.

Fig. 8B shows different compensation flow rates for different stages as compared with the fixed compensation flow rate. The solid line 804 represents the air flow rate of pumping in where the electromagnetic valve opens without compensation during the exhalation phase. The dash-dotted line 805 represents the air flow rate of pumping in where the electromagnetic valve opens with a fixed flow rate of 80 ml/second during the exhalation phase. The solid and dash-dashed lines are the same as Fig. 5B. The dashed lines 806-1, 806-2, and 806-3 represent different compensation flow rates for three stages of the exhalation phase. According to the equation (5), the compensation flow rate for Stage 1 is 20 ml/second, the compensation flow rate 806-2 for Stage 2 is 86.67 ml/second, and the compensation flow rate 806-3 for Stage 3 is 100 ml/s.

In some embodiments, the compensation flow rates for the multiple stages may be determined to be in an increasing order over time, such that the airbag deflates quickly at the beginning of the exhalation phase and then slows down gradually. Such deflation is aligned with the nature breathing waveform of expiration of human. In some embodiments, the air pump does not pump air in the starting stage to maximize the deflation rate of the airbag, for example, when the BPM is high. In some embodiments, some of the compensation flow rates may be equal. For example, when the exhalation phase comprises three stages, the second stage and the third stage may have the same compensation flow rate. In addition, depending on individual breathing characteristics, the compensation flow rates may be determined to be in a decreasing order.

For the purpose of illustrating spirit and principle of the present disclosure, some specific embodiments thereof have been described above. In general, the various example embodiments may be implemented in hardware or special purpose circuits, software, logic or any combination thereof. Some aspects may be implemented in hardware, while other aspects may be implemented in firmware or software which may be executed by a controller, microprocessor or other computing device. While various aspects of the example embodiments of the present disclosure are illustrated and described as block diagrams, flowcharts, or using some other pictorial representation, it will be appreciated that the blocks, apparatus, systems, techniques or methods described herein may be implemented in, as non-limiting examples, hardware, software, firmware, special purpose circuits or logic, general purpose hardware or controller or other computing devices, or some combination thereof.

In the context of the present disclosure, a machine readable medium may be any tangible medium that can contain, or store a program for use by or in connection with an instruction execution system, apparatus, or device. The machine readable medium may be a machine readable signal medium or a machine readable storage medium. A machine readable medium may include but not limited to an electronic, magnetic, optical, electromagnetic, infrared, or semiconductor system, apparatus, or device, or any suitable combination of the foregoing. More specific examples of the machine readable storage medium would include an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing.

Computer program code for carrying out methods of the present disclosure may be written in any combination of one or more programming languages. These computer program codes may be provided to a processor of a general purpose computer, special purpose computer, or other programmable data processing apparatus, such that the program codes, when executed by the processor of the computer or other programmable data processing apparatus, cause the functions or operations specified in the flowcharts and/or block diagrams to be implemented. The program code may execute entirely on a computer, partly on the computer, as a stand-alone software package, partly on the computer and partly on a remote computer or entirely on the remote computer or server.

Further, while operations are depicted in a particular order, this should not be understood as requiring that such operations be performed in the particular order shown or in sequential order, or that all illustrated operations be performed, to achieve desirable results. In certain circumstances, multitasking and parallel processing may be advantageous. Likewise, while several specific implementation details are contained in the above discussions, these should not be construed as limitations on the scope of any disclosure or of what may be claimed, but rather as descriptions of features that may be specific to particular embodiments of particular disclosures. Certain features that are described in this specification in the context of separate embodiments can also be implemented in combination in a single embodiment. Conversely, various features that are described in the context of a single embodiment can also be implemented in multiple embodiments separately or in any suitable sub-combination.

Various modifications, adaptations to the foregoing example embodiments of this disclosure may become apparent to those skilled in the relevant arts in view of the foregoing description, when read in conjunction with the accompanying drawings. Any and all modifications will still fall within the scope of the non-limiting and example embodiments of this disclosure. Furthermore, other embodiments of the disclosures set forth herein will come to mind to one skilled in the art to which these embodiments of the disclosure pertain having the benefit of the teachings presented in the foregoing descriptions and the drawings.

Therefore, it will be appreciated that the embodiments of the disclosure are not to be limited to the specific embodiments disclosed and that modifications and other embodiments are intended to be included within the scope of the appended claims. Although specific terms are used in the description, they are used in a generic and descriptive sense only and not for purposes of limitation, the invention being defined by the appended claims.

## Claims

1.   A method (300) for controlling a paced breathing device (100), comprising:

   determining (310) a flow rate for pumping air into an airbag (102) of the paced breathing device (100) in an exhalation phase (206) of the paced breathing device (100); and
   pumping (320), at the determined flow rate, the air into the airbag (102) in the exhalation phase (206) during which the airbag (102) releases the air in order to control the exhalation time length of the exhalation phase.

2.   The method (300) of claim 1, wherein the flow rate includes at least one compensation flow rate, and determining (310) the at least one compensation flow rate comprises:

   obtaining (410) an inflation flow rate for pumping the air into the airbag (102) in an inhalation phase (202) of the paced breathing device (100); and
   determining (420) the at least one compensation flow rate based on the inflation flow rate, a deflation flow rate at which the airbag (102) releases the air in the exhalation phase, and breathing parameters.

3.   The method (300) of claim 2, wherein the breathing parameters include breaths per minute (BPM) for the paced breathing device and user breathing parameters, and the user breathing parameters include an inhalation hold time length, an exhalation hold time length and an exhalation-to-inhalation ratio, and determining (420) the at least one compensation flow rate comprises:

   determining (422) a time length of a breathing cycle (200) based on the BPM;
   determining (424) an inhalation time length of the inhalation phase (202) and an exhalation time length of the exhalation phase (206) based on the time length of the breathing cycle (200), the exhalation-to-inhalation ratio, the inhalation hold time length and the exhalation hold time length; and
   determining (426) the at least one compensation flow rate based on the inhalation time length, the inflation flow rate, the exhalation time length and the deflation flow rate.

4.   The method (300) of claim 3, wherein the exhalation phase includes N stages (803-1, 803-2, 803-3) over time, where N is an integer greater than or equal to 2, and determining the at least one compensation flow rate further comprises: determining a compensation flow rate for each stage of the N stages (803-1, 803-2, 803-3) respectively.

5.   The method (300) of claim 4, further comprising:
   dividing (710) the exhalation phase (206) into the N stages (803-1, 803-2, 803-3) based on user breathing characteristics.

6. The method (300) of claim 4, wherein the user breathing parameters further include a first ratio indicating a proportion of a time length of a stage of the N stages (803-1, 803-2, 803-3) to the exhalation time length of the exhalation phase (206), and a second ratio indicating a proportion of volume to be released in the airbag during the stage to volume to be released in the airbag during the exhalation phase (206), and determining a compensation flow rate for each stage of the N stages (803-1, 803-2, 803-3) respectively comprises:
determining (730) a compensation flow rate (806-1, 806-2, 806-3) for each stage of the N stages (803-1, 803-2, 803-3) based on the inhalation time length, the inflation flow rate, the exhalation time length, the deflation flow rate, the first ratio and the second ratio.

7. The method (300) of any of claims 3-6, further comprising:
determining the user breathing parameters based on one or more of:

   a user interaction with the paced breathing device (100), and
   a predefined setting of the paced breathing device (100) according to an analysis of historical breathing characteristics of multiple users.

8. The method (300) of claim 1, further comprising:
adjusting a duty cycle of Power Width Modulation (PWM) of an actuator (104) of the paced breathing device (100) to enable the determined flow rate.

9. A paced breathing device (100), comprising:

   an airbag (102);
   an actuator (104) for increasing the air volume in the airbag;
   a processor (109); and
   a memory (110) having a plurality of instructions stored thereon, when executed by the processor, cause the device (100) to:

      determine (310) a flow rate for pumping air into the airbag (102) in an exhalation phase (206) of the paced breathing device (100); and
      pump (320), at the determined flow rate, the air into the airbag (102) in the exhalation phase (206) during which the airbag (102) releases the air.

10. The device (100) of claim 9, wherein the flow rate includes at least one compensation flow rate, and the instructions, when executed, further cause the device (100) to:

    obtain (410) an inflation flow rate for pumping the air into the airbag (102) in an inhalation phase (202) of the paced breathing device (100); and
    determine (420) the at least one compensation flow rate based on the inflation flow rate, a deflation flow rate at which the airbag (102) releases the air in the exhalation phase, and breathing parameters.

11. The device (100) of claim 10, wherein the breathing parameters include breaths per minute (BPM) for the paced breathing device and user breathing parameters, and the user breathing parameters include an inhalation hold time length, an exhalation hold time length, and an exhalation-to-inhalation ratio, and the instructions, when executed, further cause the device(100) to:

    determine (422) a time length of a breathing cycle (200) based on the BPM;
    determine (424) an inhalation time length of the inhalation phase (202) and an exhalation time length of the exhalation phase (206) based on the time length of the breathing cycle (200), the exhalation-to-inhalation ratio, the inhalation hold time length and the exhalation hold time length; and
    determine (426) the at least one compensation flow rate based on the inhalation time length, the inflation flow rate, the exhalation time length and the deflation flow rate.

12. The device (100) of claim 11, wherein the exhalation phase includes N stages (803-1, 803-2, 803-3) over time, where N is an integer greater than or equal to 2, and the instructions, when executed, further cause the device (100) to:
determine a compensation flow rate for each stage of the N stages (803-1, 803-2, 803-3) respectively.

13. The device (100) of claim 12, wherein the instructions, when executed, further cause the device (100) to:

divide (710) the exhalation phase (206) into the N stages (803-1, 803-2, 803-3) based on user breathing characteristics.

14. The device (100) of claim 12, wherein the user breathing parameters further include a first ratio indicating a proportion of a time length of a stage of the N stages (803-1, 803-2, 803-3) to the exhalation time length of the exhalation phase (206), and a second ratio indicating a proportion of volume to be released in the airbag during the stage to volume to be released in the airbag during the exhalation phase (206), and the instructions, when executed, further cause the device (100) to:
determine (730) a compensation flow rate for each stage of the N stages (803-1, 803-2, 803-3) based on the inhalation time length, the inflation flow rate, the exhalation time length, the deflation flow rate, the first ratio and the second ratio.

15. A computer program product comprising a computer readable medium, the computer readable medium having computer readable code embodied therein, the computer readable code being configured such that, on execution by the processor (109) of the paced breathing device (100) according to any one of claims 9-14, the paced breathing device (100) is caused to perform the method as claimed in any one of claims 1 to 8.

## Patentansprüche

1. Verfahren (300) zum Steuern einer stimulierten Beatmungsvorrichtung (100), umfassend:

   Bestimmen (310) einer Flussrate zum Pumpen von Luft in ein Luftkissen (102) der stimulierten Beatmungsvorrichtung (100) in einer Ausatmungsphase (206) der stimulierten Beatmungsvorrichtung (100); und
   Pumpen (320) der Luft mit der bestimmten Flussrate in das Luftkissen (102) in der Ausatmungsphase (206), in der das Luftkissen (102) die Luft freigibt, um die Ausatmungszeitdauer der Ausatmungsphase zu steuern.

2. Verfahren (300) nach Anspruch 1, wobei die Flussrate mindestens eine Kompensationsflussrate beinhaltet und Bestimmen (310) der mindestens einen Kompensationsflussrate umfasst:

   Erhalten (410) einer Aufblasflussrate zum Pumpen der Luft in das Luftkissen (102) in einer Einatmungsphase (202) der stimulierten Beatmungsvorrichtung (100); und
   Bestimmen (420) der mindestens einen Kompensationsflussrate basierend auf der Aufblasflussrate, einer Ablassflussrate, bei der das Luftkissen (102) die Luft in der Ausatmungsphase freigibt, und Atemparametern.

3. Verfahren (300) nach Anspruch 2, wobei die Atemparameter Atemzüge pro Minute (BPM) für die stimulierte Beatmungsvorrichtung und Benutzer-Atemparameter beinhalten, und die Benutzer-Atemparameter eine Einatmungshaltezeitdauer, eine Ausatmungshaltezeitdauer und ein Verhältnis von Ausatmung zu Einatmung beinhalten, und Bestimmen (420) der mindestens einen Kompensationsflussrate umfasst:

   Bestimmen (422) einer Zeitdauer eines Atemzyklus (200) basierend auf den BPM;
   Bestimmen (424) einer Einatmungszeitdauer der Einatmungsphase (202) und einer Ausatmungszeitdauer der Ausatmungsphase (206) basierend auf der Zeitdauer des Atemzyklus (200), dem Verhältnis von Ausatmung zu Einatmung, der Einatmungshaltezeitdauer und der Ausatmungshaltezeitdauer; und
   Bestimmen (426) der mindestens einen Kompensationsflussrate basierend auf der Einatmungszeitdauer, der Aufblasflussrate, der Ausatmungszeitdauer und der Ablassflussrate.

4. Verfahren (300) nach Anspruch 3, wobei die Ausatmungsphase N Stufen (803-1, 803-2, 803-3) über die Zeit hinweg beinhaltet, wobei N eine Ganzzahl größer oder gleich 2 ist, und Bestimmen der mindestens einen Kompensationsflussrate weiter umfasst:
Bestimmen einer Kompensationsflussrate jeweils für jede Stufe der N Stufen (803-1, 803-2, 803-3).

5. Verfahren (300) nach Anspruch 4, weiter umfassend:
Aufteilen (710) der Ausatmungsphase (206) in die N Stufen (803-1, 803-2, 803-3) basierend auf Atemeigenschaften des Benutzers.

6. Verfahren (300) nach Anspruch 4, wobei die Atemparameter des Benutzers weiter ein erstes Verhältnis beinhalten, das einen Anteil einer Zeitdauer einer Stufe der N Stufen (803-1, 803-2, 803-3) zur Ausatmungszeitdauer der Ausatmungsphase (206) angibt, und ein zweites Verhältnis, das einen Anteil eines Volumens angibt, das während der Stufe, um Volumen in das Luftkissen freizugeben, während der Ausatmungsphase (206) freizugeben ist, und Bestimmen einer Kompensationsflussrate für jede Stufe der N Stufen (803-1, 803-2, 803-3) jeweils umfasst: Bestimmen (730) einer Kompensationsflussrate (806-1, 806-2, 806-3) für jede Stufe der N Stufen (803-1, 803-2, 803-3) basierend auf der Einatmungszeitdauer, der Aufblasflussrate, der Ausatmungszeitdauer, der Ablassflussrate, dem ersten Verhältnis und dem zweiten Verhältnis.

7. Verfahren (300) nach einem der Ansprüche 3-6, weiter umfassend:
Bestimmen der Atemparameter des Benutzers basierend auf einem oder mehr von:

einer Benutzerinteraktion mit der stimulierten Beatmungsvorrichtung (100), und
einer vordefinierten Einstellung der stimulierten Beatmungsvorrichtung (100) gemäß einer Analyse historischer Atemeigenschaften mehrerer Benutzer.

8. Verfahren (300) nach Anspruch 1, weiter umfassend:
Anpassen eines Arbeitszyklus einer Leistungsweitenmodulation (PWM) einer Betätigungsvorrichtung (104) der stimulierten Beatmungsvorrichtung (100), um die bestimmte Flussrate zu ermöglichen.

9. Stimulierte Beatmungsvorrichtung (100), umfassend:

ein Luftkissen (102);
eine Betätigungsvorrichtung (104) zum Erhöhen des Luftvolumens in dem Luftkissen;
einen Prozessor (109); und
einen Speicher (110), der eine Vielzahl von darauf gespeicherten Anweisungen aufweist, die, wenn sie vom Prozessor ausgeführt werden, die Vorrichtung (100) veranlassen:

eine Flussrate zum Pumpen von Luft in das Luftkissen (102) in einer Ausatmungsphase (206) der stimulierten Beatmungsvorrichtung (100) zu bestimmen (310); und
die Luft mit der bestimmten Flussrate in das Luftkissen (102) in der Ausatmungsphase (206) zu pumpen (320), in der das Luftkissen (102) die Luft freigibt.

10. Vorrichtung (100) nach Anspruch 9, wobei die Flussrate mindestens eine Kompensationsflussrate beinhaltet, und die Anweisungen, wenn sie ausgeführt werden, die Vorrichtung (100) weiter veranlassen:

eine Aufblasflussrate zum Pumpen der Luft in das Luftkissen (102) in einer Einatmungsphase (202) der stimulierten Beatmungsvorrichtung (100) zu erhalten (410); und
die mindestens eine Kompensationsflussrate basierend auf der Aufblasflussrate, einer Ablassflussrate, bei der das Luftkissen (102) die Luft in der Ausatmungsphase freigibt, und Atemparametern zu bestimmen (420).

11. Vorrichtung (100) nach Anspruch 10, wobei die Atemparameter Atemzüge pro Minute (BPM) für die stimulierte Beatmungsvorrichtung und Benutzer-Atemparameter beinhalten, und die Benutzer-Atemparameter eine Einatmungshaltezeitdauer, eine Ausatmungshaltezeitdauer und ein Verhältnis von Ausatmung zu Einatmung beinhalten, und die Anweisungen, wenn sie ausgeführt werden, die Vorrichtung (100) weiter veranlassen:

eine Zeitdauer eines Atemzyklus (200) basierend auf den BPM zu bestimmen (422);
eine Einatmungszeitdauer der Einatmungsphase (202) und eine Ausatmungszeitdauer der Ausatmungsphase (206) basierend auf der Zeitdauer des Atemzyklus (200), dem Verhältnis von Ausatmung zu Einatmung, der Einatmungshaltezeitdauer und der Ausatmungshaltezeitdauer zu bestimmen (424); und
die mindestens eine Kompensationsflussrate basierend auf der Einatmungszeitdauer, der Aufblasflussrate, der Ausatmungszeitdauer und der Ablassflussrate zu bestimmen (426).

12. Vorrichtung (100) nach Anspruch 11, wobei die Ausatmungsphase N Stufen (803-1, 803-2, 803-3) über die Zeit hinweg beinhaltet, wobei N eine Ganzzahl größer oder gleich 2 ist, und die Anweisungen, wenn sie ausgeführt werden, die Vorrichtung (100) weiter veranlassen:
jeweils eine Kompensationsflussrate für jede Stufe der N Stufen (803-1, 803-2, 803-3) zu bestimmen.

13. Vorrichtung (100) nach Anspruch 12, wobei die Anweisungen, wenn sie ausgeführt werden, die Vorrichtung (100) weiter veranlassen:
die Ausatmungsphase (206) in die N Stufen (803-1, 803-2, 803-3) basierend auf Atemeigenschaften des Benutzers aufzuteilen (710).

14. Vorrichtung (100) nach Anspruch 12, wobei die Atemparameter des Benutzers weiter ein erstes Verhältnis beinhalten, das einen Anteil einer Zeitdauer einer Stufe der N Stufen (803-1, 803-2, 803-3) zur Ausatmungszeitdauer der Ausatmungsphase (206) angibt, und ein zweites Verhältnis, das einen Anteil eines Volumens angibt, das während der Stufe, um Volumen in das Luftkissen freizugeben, während der Ausatmungsphase (206) freizugeben ist, und die Anweisungen, wenn sie ausgeführt werden, die Vorrichtung (100) weiter veranlassen:
eine Kompensationsflussrate für jede Stufe der N Stufen (803-1, 803-2, 803-3) basierend auf der Einatmungszeitdauer, der Aufblasflussrate, der Ausatmungszeitdauer, der Ablassflussrate, dem ersten Verhältnis und dem zweiten Verhältnis zu bestimmen (730).

15. Computerprogrammprodukt, das ein computerlesbares Medium umfasst, wobei das computerlesbare Medium einen darin integrierten computerlesbaren Code aufweist, wobei der computerlesbare Code konfiguriert ist, sodass bei Ausführung durch den Prozessor (109) der stimulierten Beatmungsvorrichtung (100) nach einem der Ansprüche 9-14 die stimulierte Beatmungsvorrichtung (100) veranlasst wird, das Verfahren nach einem der Ansprüche 1 bis 8 durchzuführen.

**Revendications**

1. Procédé (300) de commande d'un dispositif de respiration stimulée (100), comprenant :

   la détermination (310) d'un débit pour pomper de l'air dans un coussin gonflable (102) du dispositif de respiration stimulée (100) dans une phase d'expiration (206) du dispositif de respiration stimulée (100) ; et
   le pompage (320), au débit déterminé, de l'air dans le coussin gonflable (102) dans la phase d'expiration (206) pendant laquelle le coussin gonflable (102) libère l'air afin de commander la durée d'expiration de la phase d'expiration.

2. Procédé (300) selon la revendication 1, dans lequel le débit inclut au moins un débit de compensation, et la détermination (310) du au moins un débit de compensation comprend :

   l'obtention (410) d'un débit de gonflage pour pomper l'air dans le coussin gonflable (102) dans une phase d'inhalation (202) du dispositif de respiration stimulée (100) ; et
   la détermination (420) du au moins un débit de compensation sur la base du débit de gonflage, d'un débit de dégonflage auquel le coussin gonflable (102) libère l'air dans la phase d'expiration, et de paramètres respiratoires.

3. Procédé (300) selon la revendication 2, dans lequel les paramètres respiratoires incluent des respirations par minute (RPM) pour le dispositif de respiration stimulée et des paramètres respiratoires de l'utilisateur, et les paramètres respiratoires de l'utilisateur incluent une durée de maintien de l'inspiration, une durée de maintien de l'expiration et un rapport expiration/inspiration, et la détermination (420) du au moins un débit de compensation comprend :

   la détermination (422) d'une durée d'un cycle respiratoire (200) sur la base des RPM ;
   la détermination (424) d'une durée d'inspiration de la phase d'inspiration (202) et d'une durée d'expiration de la phase d'expiration (206) sur la base de la durée du cycle respiratoire (200), du rapport expiration/inspiration, de la durée de maintien de l'inspiration et de la durée de maintien de l'expiration ; et
   la détermination (426) du au moins un débit de compensation sur la base de la durée d'inspiration, du débit de gonflage, de la durée d'expiration et du débit de dégonflage.

4. Procédé (300) selon la revendication 3, dans lequel la phase d'expiration inclut N étapes (803-1, 803-2, 803-3) au fil du temps, où N est un entier supérieur ou égal à 2, et la détermination du au moins un débit de compensation comprend en outre :
la détermination d'un débit de compensation pour chaque étape des étapes N (803-1, 803-2, 803-3) respectivement.

5. Procédé (300) selon la revendication 4, comprenant en outre :
la division (710) de la phase d'expiration (206) en N étapes (803-1, 803-2, 803-3) sur la base de caractéristiques respiratoires de l'utilisateur.

**6.** Procédé (300) selon la revendication 4, dans lequel les paramètres respiratoires de l'utilisateur incluent en outre un premier rapport indiquant une proportion d'une durée d'une étape des N étapes (803-1, 803-2, 803-3) par rapport à la durée d'expiration de la phase d'expiration (206), et un second rapport indiquant une proportion de volume à libérer dans le coussin gonflable pendant l'étape par rapport au volume à libérer dans le coussin gonflable pendant la phase d'expiration (206), et la détermination d'un débit de compensation pour chaque étape des N étapes (803-1, 803-2, 803-3) comprend respectivement :
la détermination (730) d'un débit de compensation (806-1, 806-2, 806-3) pour chaque étape des N étapes (803-1, 803-2, 803-3) sur la base de la durée d'inspiration, du débit de gonflage, de la durée d'expiration, du débit de dégonflage, du premier rapport et du second rapport.

**7.** Procédé (300) selon l'une quelconque des revendications 3-6, comprenant en outre :
la détermination des paramètres respiratoires de l'utilisateur sur la base d'un ou de plusieurs parmi :

une interaction de l'utilisateur avec le dispositif de respiration stimulée (100), et
un réglage prédéfini du dispositif de respiration stimulée (100) en fonction d'une analyse de caractéristiques respiratoires historiques de plusieurs utilisateurs.

**8.** Procédé (300) selon la revendication 1, comprenant en outre :
le réglage d'un cycle de service de modulation de largeur de puissance (PWM) d'un actionneur (104) du dispositif de respiration stimulée (100) pour permettre le débit déterminé.

**9.** Dispositif de respiration stimulée (100), comprenant :

un coussin gonflable (102) ;
un actionneur (104) pour augmenter le volume d'air dans le coussin gonflable ;
un processeur (109) ; et
une mémoire (110) présentant une pluralité d'instructions stockées sur celle-ci qui, lorsqu'elles sont exécutées par le processeur, amènent le dispositif (100) à :

déterminer (310) un débit pour pomper de l'air dans le coussin gonflable (102) dans une phase d'expiration (206) du dispositif de respiration stimulée (100) ; et
pomper (320), au débit déterminé, l'air dans le coussin gonflable (102) dans la phase d'expiration (206) pendant laquelle le coussin gonflable (102) libère l'air.

**10.** Dispositif (100) selon la revendication 9, dans lequel le débit inclut au moins un débit de compensation, et les instructions, lorsqu'elles sont exécutées, amènent en outre le dispositif (100) à :

obtenir (410) un débit de gonflage pour pomper l'air dans le coussin gonflable (102) dans une phase d'inhalation (202) du dispositif de respiration stimulée (100) ; et
déterminer (420) le au moins un débit de compensation sur la base du débit de gonflage, d'un débit de dégonflage auquel le coussin gonflable (102) libère l'air dans la phase d'expiration, et de paramètres respiratoires.

**11.** Dispositif (100) selon la revendication 10, dans lequel les paramètres respiratoires incluent les respirations par minute (RPM) pour le dispositif de respiration stimulée et des paramètres respiratoires de l'utilisateur, et les paramètres respiratoires de l'utilisateur incluent une durée de maintien de l'inspiration, une durée de maintien de l'expiration et un rapport expiration/inspiration, et les instructions, lorsqu'elles sont exécutées, amènent en outre le dispositif (100) à :

déterminer (422) une durée d'un cycle respiratoire (200) sur la base des RPM ;
déterminer (424) une durée d'inspiration de la phase d'inspiration (202) et une durée d'expiration de la phase d'expiration (206) sur la base de la durée du cycle respiratoire (200), du rapport expiration/inspiration, de la durée de maintien de l'inspiration et de la durée de maintien de l'expiration ; et
déterminer (426) le au moins un débit de compensation sur la base de la durée d'inspiration, du débit de gonflage, de la durée d'expiration et du débit de dégonflage.

**12.** Dispositif (100) selon la revendication 11, dans lequel la phase d'expiration inclut N étapes (803-1, 803-2, 803-3) au fil du temps, où N est un entier supérieur ou égal à 2, et les instructions, lorsqu'elles sont exécutées, amènent en outre le dispositif (100) à :
déterminer un débit de compensation pour chaque étape des N étapes (803-1, 803-2, 803-3) respectivement.

**13.** Dispositif (100) selon la revendication 12, dans lequel les instructions, lorsqu'elles sont exécutées, amènent en outre le dispositif (100) à :
diviser (710) la phase d'expiration (206) en N étapes (803-1, 803-2, 803-3) sur la base de caractéristiques respiratoires de l'utilisateur.

**14.** Dispositif (100) selon la revendication 12, dans lequel les paramètres respiratoires de l'utilisateur incluent en outre un premier rapport indiquant une proportion d'une durée d'une étape des N étapes (803-1, 803-2, 803-3) par rapport à la durée d'expiration de la phase d'expiration (206), et un second rapport indiquant une proportion de volume à libérer dans le coussin gonflable pendant l'étape par rapport au volume à libérer dans le coussin gonflable pendant la phase d'expiration (206), et les instructions, lorsqu'elles sont exécutées, amènent en outre le dispositif (100) à :
déterminer (730) un débit de compensation pour chaque étape des N étapes (803-1, 803-2, 803-3) sur la base de la durée d'inspiration, du débit de gonflage, de la durée d'expiration, du débit de dégonflage, du premier rapport et du second rapport.

**15.** Produit programme informatique comprenant un support lisible par ordinateur, le support lisible par ordinateur présentant un code lisible par ordinateur incorporé dans celui-ci, le code lisible par ordinateur étant configuré de telle sorte que, lors de son exécution par le processeur (109) du dispositif de respiration stimulée (100) selon l'une quelconque des revendications 9-14, le dispositif de respiration stimulée (100) soit amené à réaliser le procédé selon l'une quelconque des revendications 1 à 8.

100

104

ACTUATOR

AIRBAG

102

108

CONTROL UNIT

| PROCESSOR | MEMORY |

VALVE

106

109

110

**FIG. 1**

FIG. 2

300

310

DETERMINE A FLOW RATE FOR PUMPING AIR INTO AN AIRBAG OF THE PACED BREATHING DEVICE IN AN EXHALATION PHASE OF THE PACED BREATHING DEVICE

320

PUMP, AT THE DETERMINED FLOW RATE, THE AIR INTO THE AIRBAG IN THE EXHALATION PHASE DURING WHICH THE AIRBAG RELEASES THE AIR

**FIG. 3**

400

┌─ 410
OBTAIN AN INFLATION FLOW RATE FOR PUMPING THE AIR INTO THE AIRBAG IN AN
INHALATION PHASE OF THE PACED BREATHING DEVICE

┌─ 420
DETERMINE THE COMPENSATION FLOW RATE BASED ON THE INFLATION FLOW RATE,
A DEFLATION FLOW RATE AT WHICH THE AIRBAG RELEASES THE AIR, AND
BREATHING PARAMETERS

┌─ 422
DETERMINE A TIME LENGTH OF A BREATHING CYCLE BASED ON THE BPM

┌─ 424
DETERMINE AN INHALATION TIME LENGTH OF THE INHALATION PHASE AND AN
EXHALATION TIME LENGTH OF THE EXHALATION PHASE BASED ON THE TIME
LENGTH OF THE BREATHING CYCLE, THE EXHALATION-TO-INHALATION RATIO, THE
INHALATION HOLD TIME LENGTH AND THE EXHALATION HOLD TIME LENGTH

┌─ 426
DETERMINE THE COMPENSATION FLOW RATE BASED ON THE INHALATION TIME
LENGTH, THE INFLATION FLOW RATE, THE EXHALATION TIME LENGTH AND THE
DEFLATION FLOW RATE

**FIG. 4**

FIG. 5A

**FIG. 5B**

**FIG. 6A**

simulation result of air flow rate of pumping in during deflation

FIG. 6B

700 ⟍

```
                                                        ⌐710
┌──────────────────────────────────────────────────────────┐
│    DIVIDE THE EXHALATION PHASE INTO N STAGES BASED ON USER │
│                 BREATHING CHARACTERISTICS                  │
└──────────────────────────────────────────────────────────┘
                              │
                              ▼
                                                        ⌐720
┌──────────────────────────────────────────────────────────┐
│    DETERMINE A  FIRST RATIO AND A SECOND RATIO FOR EACH STAGE │
└──────────────────────────────────────────────────────────┘
                              │
                              ▼
                                                        ⌐730
┌──────────────────────────────────────────────────────────┐
│   DETERMINE A COMPSENATION FLOW RATE FOR EACH STAGE  BASED │
│   ON  THE INHALATION TIME LENGTH, THE INFLATION FLOW RATE, THE │
│   EXHALATION TIME LENGTH, THE DEFLATION FLOW RATE, THE FIRST │
│                 RATIO AND THE SECOND RATIO                 │
└──────────────────────────────────────────────────────────┘
```

**FIG. 7**

**FIG. 8A**

**FIG. 8B**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113926157 A